# EUROPEAN PATENT APPLICATION

(11) **EP 4 728 988 A1**
(43) Date of publication of application: **22.04.2026**
(21) Application number: 24383157.5
(22) Date of filing: 21.10.2024
(51) Int. Cl.: A61B 5/346, A61B 5/00

(54) **METHOD AND SYSTEM FOR OBTAINING ECG DATA AND TO DETECT AND FILTER LEFT VENTRICULAR ASSIST DEVICE INTERFERENCES**

(71) Applicant: Vision to AI S.L., 28003 Madrid (ES); Universidad Complutense De Madrid, 28040 Madrid (ES); Fundación para la Investigación Biomédica del Hospital Universitario Ramón y Cajal, 28034 Madrid (ES); Fundación Pública Miguel Servet - Navarrabiomed, 31008 Pamplona (ES)
(72) Inventor: Alonso Salinas, Gonzalo Luis, Pamplona (ES); Buelga Suárez, Mauro, Madrid (ES); Piñuel Moreno, Luis, Madrid (ES); Recas Piorno, Joaquín, Madrid (ES); Diaz Vicente, Marian, Madrid (ES)
(74) Representative: Pons IP

(57) **Abstract**

Method for obtaining ECG data, determining if it contains noise patterns from an external assistance device like a Left Ventricular Assist Device (LVAD) and dynamically filtering them. The method comprises the steps of obtaining ECG data from a patient; and checking if the ECG data comprises noise patterns from the external assistance device by analyzing the ECG data using a time and frequency transformation and classifying the analyzed ECG data as with or without LVAD noise patterns, and if the ECG data comprises noise patterns: applying a Fast Fourier Transform for transforming the ECG data into a frequency domain; clustering the ECG data by frequency; searching for a principal rotation frequency and secondary rotation frequencies of the pump; filtering the principal and the secondary rotation frequencies from the ECG data; and obtaining filtered ECG data, and if the ECG data does not comprise noise patterns: outputting the obtained ECG data.

## Description

### OBJECT OF THE INVENTION

The invention is related to the field of electrocardiogram (ECG) data obtention, more particularly to ECG data obtained from patients with external assistance devices.

The object of the invention is a method for obtaining ECG data which allows to check and filter the interferences introduced by external assistance devices.

### BACKGROUND ART

A Left Ventricular Assist Device (LVAD) is an assistance device intended for providing circulatory support to patients with advanced heart failure, e.g. with ischemic cardiopathies on a transplant waiting list. The LVAD is used until reversible contraindications for a transplant are solved, until the heart recovers or as chronic and palliative therapy in these patients.

The LVAD acts as an artificial left ventricle by being placed at the apex of the left ventricle, in the mitral valve, towards the aortic artery. The LVAD comprises a pump, which is a continuous axial flow magnetic levitation system.

The pump guarantees cardiac output when the heart cannot be properly contracted and generates a low output.

Nevertheless, LVADs generate electromagnetic interferences that gives rise to noise artifacts in conventional ECGs that prevent accurate clinical analysis.

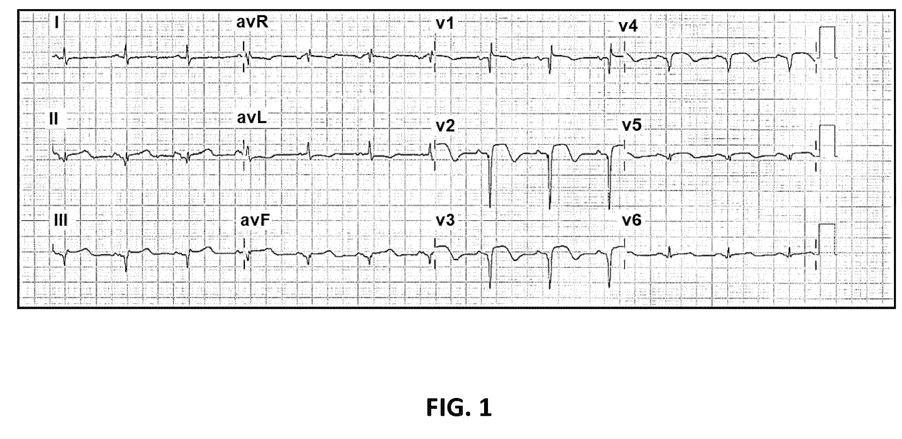

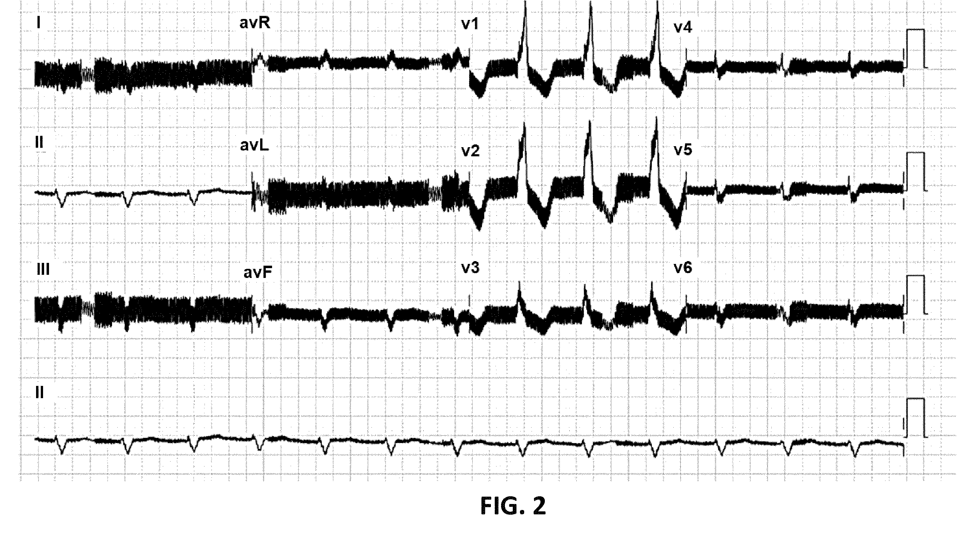

For example, Figure 1 shows an ECG without noise artifacts and Figure 2 shows an ECG of patient with LVAD in which a low-pass filter at 150 Hz and a high-pass filter at 0.05 Hz have been applied. In the ECG of Figure 2 it could be seen that the pump rotation introduces noise artifacts.

The LVAD typically operate at three different frequencies. The pump rotates at a principal rotation frequency. Then, it slows down to a secondary rotation frequency and speeds up to another secondary rotation frequency and then slows down again to rotate at the principal rotation frequency. In an example, a pump rotating at 4000rpm would lower the rotation to 2000rpm and then would speed up to 6000rpm reducing its speed again to 4000rpm. This operation allows to imitate the heart operation (beats) reducing the damage on the veins and arteries and avoiding stasis.

A protocol has been developed to the conventional ECG stating that they should be configured with a 150 Hz low-pass filter and a 0.05 Hz high-pass filter.

Nevertheless, current commercial electrocardiographs can only lower the low-pass filter up to 40 Hz. Even in this minimal configuration, the noise artifacts are present, also, setting the low-pass filter out of the recommendations in the protocol would lead to inaccurate measures.

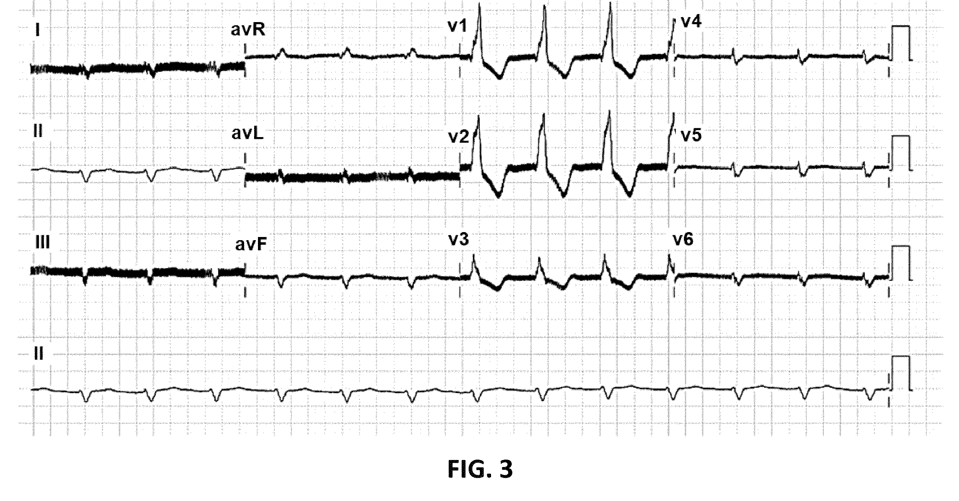

Figure 3 shows the same ECG than in Figure 3 but modifying the band of the bandpass filter at 0.05 Hz - 40 Hz.

LVADs are a relatively recent technology, so the available scientific research is limited. Moreover, in addition to the problems in the interpretation of ECGs, the LVADs introduce more handicaps, since it is a life support device which is controlled exclusively by information provided by the device itself. Also, it allows poor individualization since it does not allow to obtain more performance information.

### DESCRIPTION OF THE INVENTION

The present invention discloses a method for obtaining electrocardiogram (ECG) data which allows to check if any distortion is introduced by external assistance devices, specially a Left Ventricular Assist Device (LVAD), while maintaining the electric information from the patient heart.

The method of the invention comprises the steps of:
- obtaining ECG data from a patient;
- checking if the ECG data comprises noise patterns from a Left Ventricular Assist Device (LVAD), which comprises a pump, by analyzing the obtained ECG data using a time and frequency transformation and classifying the analyzed ECG data as with or without LVAD noise patterns, and if the ECG data comprises LVAD noise patterns:
   ∘ applying a frequency transformation, preferably a Fast Fourier Transform (FFT), for transforming the ECG data into a frequency domain;
   ∘ search for a principal rotation frequency of the pump in a range previously known;
   ∘ clustering the ECG data by frequency, generating one or more clusters;
   ∘ determining secondary rotation frequencies of the pump from clusters generated;
   ∘ filtering the principal rotation frequency and the secondary rotation frequencies from the ECG data; and
   ∘ outputting filtered ECG data.

If the ECG data does not comprise LVAD noise patterns, the obtained ECG data is outputted as filtered ECG data.

Preferably, the step of checking if the ECG data comprises noise patterns from the LVAD comprises the sub-steps of analyzing the ECG data in frequency and time by performing a Continuous Wavelet Transform (CWT), preferably, obtaining a scalogram, and classifying the analyzed ECG data for determining the presence of the LVAD.

In a preferred set of embodiments, the method further comprises the step of filtering also an alternate current interference, with a previously known frequency.

In the step of determining secondary rotation frequencies, the method could determine two or more secondary frequencies representative of the typical functioning of the LVADs.

For characterizing the operation of the LVAD, the method could comprise a step of determining and outputting actual operation data of the LVAD, preferably, the working frequencies and a time on each frequency. Therefore, the method of the invention would allow to analyze the performance of the LVAD in a specific patient and even set the working frequencies more precisely to said patient.

By analyzing the operation of the LVAD, also the method of the invention could determine anomalies in their operation. For example, the method of the invention could determine anomalies by comparing the principal rotation frequency and the secondary rotation frequencies obtained with a range previously known, determined for example by the manufacturer or the medical team. Thus, the method of the invention allows to check that the pump is working at the right frequency. This is of a key importance since the precise set of the rotation frequency depends on the patient corporal surface.

The filtering step could be preferably performed by using sequentially dynamic filters. More preferably, by using Notch filters with Infinite Impulse Response (IIR) filters. This allows a very specific filtering of the frequencies selected.

These filters could be set to filter alternate current interference in the range of 50Hz to 60Hz, or the range set by the law in each country.

Preferably, the step of classifying the analyzed ECG data for determining the presence of a LVAD could be performed using deep learning classification techniques or using image processing techniques. More preferably, these techniques could determine the presence of a LVAD by detecting noise patterns in the time and frequency transformation of the ECG data, preferably using the scalogram.

Additionally, the step of clustering the ECG data by frequency could further comprise a stage of selecting a maximum value of each cluster as a representative value of the secondary rotation frequencies.

The invention also relates to an ECG system which comprises:
- one or more electrodes intended to be attached to a patient and configured to obtain ECG measurements;
- an ECG module, connected to the electrodes and configured to digitalize and process ECG measurements, and
- a processing module connected to the ECG module and configured to perform the steps of the method previously defined.

In some embodiments, the processing module could be placed between the electrodes and the ECG module. In this case, the method of the invention would work with an analogic signal, producing an analogic filtered signal representative of the ECG for feeding the ECG module with a signal without artifacts.

Alternatively, the processing module could be placed after the ECG module. In this case, the method of the invention would work with a digital signal, producing a digital filtered signal representative of the ECG for being analyzed using ECG techniques known in the art.

In such cases, the processing module could be placed in a cloud server connected to the ECG module and configured to receive ECG data from said ECG module. In this case, the ECG system would not interfere with the devices of the hospital or clinic.

Also, the processing module could be placed in a mobile device connected to the ECG module and configured to receive ECG data from said ECG module.

Thus, the invention could be applied to home assistance, hospitals or clinics with less expertise analyzing ECG measurements,

To sum up, the method and system of the invention allows:
- Attenuation the electromagnetic noise produced by the LVAD minimizing distortion and loss of information of the original ECG signal;
- Automatic detection of the principal and secondary rotation frequencies of the LVAD, without needing to know its programming or additional information;
- Dynamic design of digital filters that filter the rotation frequencies of the pump and reduces the interference in the frequency band where there is information on the electrical functioning of the patient's heart;
- Precise filtering in the diagnostic band recommended by major medical associations: 0.05 Hz - 150 Hz; and
- Automatic detection of actual pump operating conditions, such as Rotational speeds, time at each speed, acceleration/braking periods, etc.

Also, the method and system of the invention allows an increased flexibility, since it works for ECG signals of any duration, at any sample rate within electrocardiogram standards, and with any number of leads.

Also, works with the different industry standards for medical information exchange (HL7, DICOM, SCP-ECG, FHIR, XML, csv, etc), thus, easing its integration with ECG management systems from any manufacturer.

Moreover, since the method has a low complexity, it can be implemented on processors with limited resources.

These features, also allows to monitor patients with LVAD in an out-of-hospital environment, e.g. implementing the method on Holter, mobile devices, long-acting implantable devices, etc.

The method of the invention also opens the possibility of determining anomalies in the operation of the pump of the LVAD. For example, it is possible to compare the actual operation of the pump with that declared by the manufacturer or to protect a patient from human device programming errors.

Moreover, the analysis of the operation of the pump allows to optimize pump operation for each patient, leading to a personalized medicine.

### DESCRIPTION OF THE DRAWINGS

Fig. 1. Shows an ECG of a patient without an LVAD
Figure 2 shows an ECG of patient with LVAD in which a low-pass filter at 150 Hz and a high-pass filter at 0.05 Hz have been applied.
Figure 3 shows the ECG of Figure 3 but modifying the band of the bandpass filter at 0.05 Hz - 40 Hz.
Figure 4 shows a diagram block of an exemplary embodiment of the method of the invention.
Figure 5 shows an example of a hardware device according to the present invention.
Figure 6 shows an exemplary embodiment of the invention with the processing module configured to apply the method in the invention placed between the electrodes and the ECG module.
Figure 7 shows an exemplary embodiment of the invention with the processing module configured to apply the method in the invention placed after the ECG module in the electrograph.
Figure 8 shows an exemplary embodiment of the invention with the processing module configured to apply the method in the invention placed after the ECG module out of the electrograph.
Figure 9 shows an exemplary embodiment of the invention with the processing module configured to apply the method in the invention placed after the ECG module in a cloud server.
Figure 10 shows an example of obtained ECG data without filtering.
Figure 11 shows an example of a scalogram of a patient with LDAV. The LVAD noise pattern can be clearly identified in the superior part of the image.
Figure 12 shows an example of the ECG data in time domain and in frequency domain.
Figure 13 shows an example of the obtained filtered ECG data in a patient with implanted pacemaker.
Figure 14 shows an example of the obtained filtered ECG data with a close view of the pacemaker's spicules.

### PREFERRED EMBODIMENTS OF THE INVENTION

The invention relates to a method for obtaining ECG data from patients with or without external assistance devices, in this case, a Left Ventricular Assist Device (LVAD).

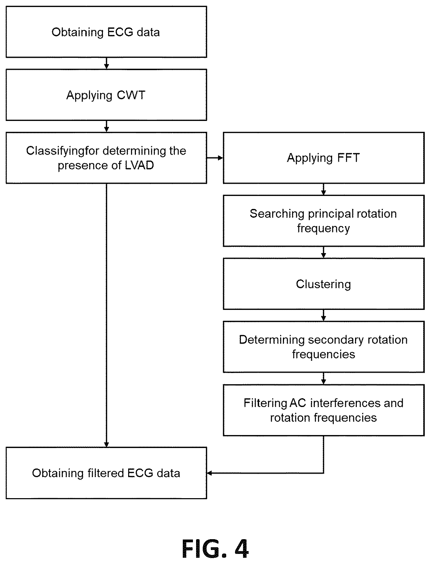

Figure 4 shows a diagram block of an exemplary embodiment of the method of the invention.

The method of the invention comprises the steps of obtaining ECG data from the patient, then, analyzing the ECG data in frequency and time by performing a time and frequency transformation, in this case, a Continuous Wavelet Transform (CWT) for obtaining a scalogram.

The scalogram, or similar time and frequency transformation of the ECG signal, is classified for determining the presence of a LVAD. Then, the ECG data is transformed into a frequency domain by applying a Fast Fourier Transform (FFT), and they are filtered eliminating the alternate current interference, the principal rotation frequency and the secondary rotation frequencies. For determining the secondary rotation frequencies, a clustering step is performed. Then, the filtered ECG data is outputted.

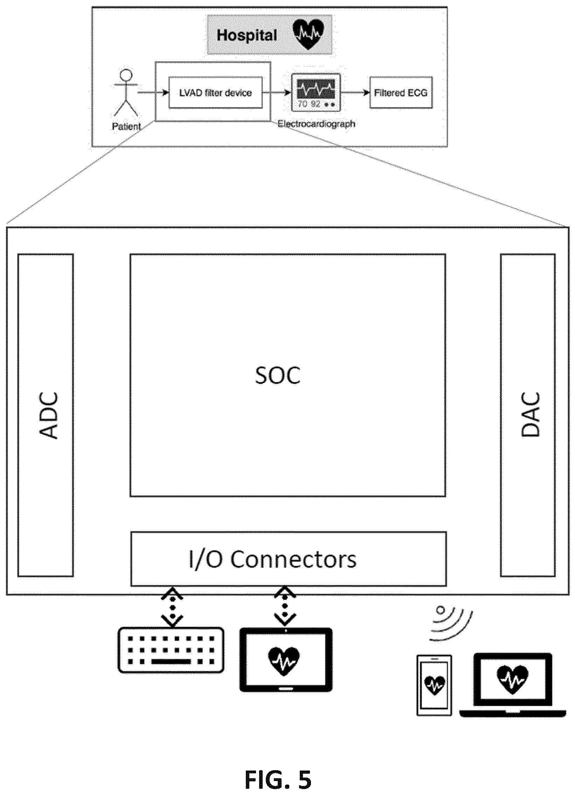

Figure 5 shows an example of a hardware device according to the present invention. In this device, the input is analog ECG data and a sampling frequency. The device is configured to apply the method described in the ECG data, outputting filtered analog ECG data, reducing the noise of the pump of the LVAD if it is present.

The device in this case comprises an analog-to-digital converter (ADC) with preferably 1 to 15 channels, a processing module, in this case a system-on-chip (SOC), and a digital-to-analog converter (DAC) with 1 to 15 channels.

Preferably, it also comprises input/output connectors:
- input connectors: configured to program the system, such as a keyboard or a wireless connection;
- output connectors: for visualizing the filtered ECG data and/or the operating conditions of the pump, such as a display, connected by a wire or wirelessly.

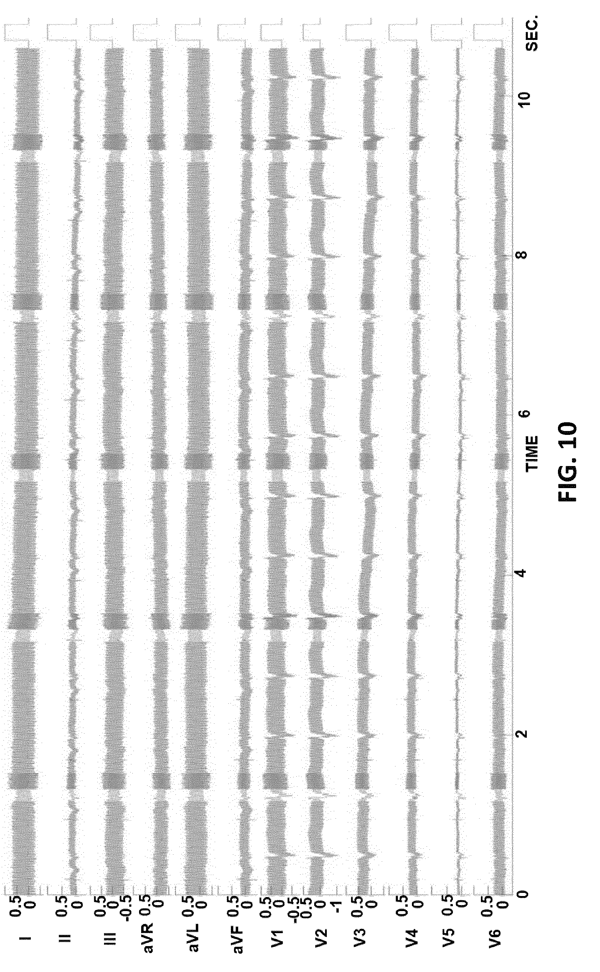

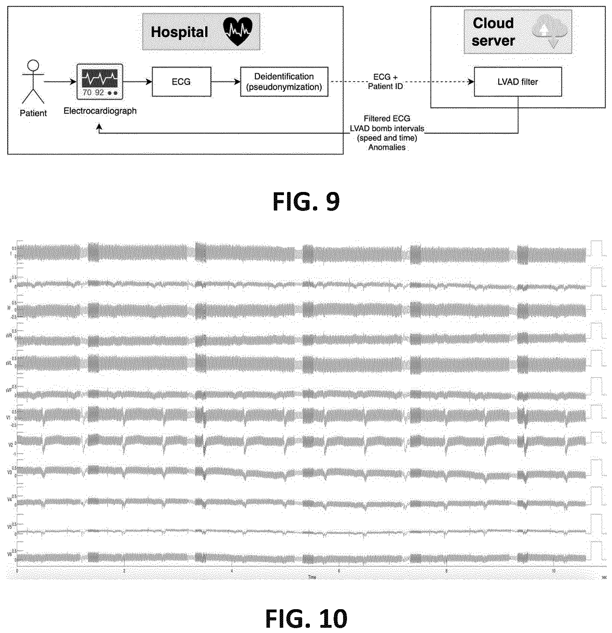

Figures 10 to 14 show an example of the results obtained by the application of the method of the invention in a patient.

The method of the invention receives as inputs a digital or analog ECG data, as shown in Figure 10. In the case of using a cloud server, the data could be anonymized by deleting personal information and identifying the ECG by an identification code.

Then, a time and frequency analysis of the ECG obtained is performed to differentiate the main frequencies of the signal according to if their origin is the LVAD or the patient's heart.

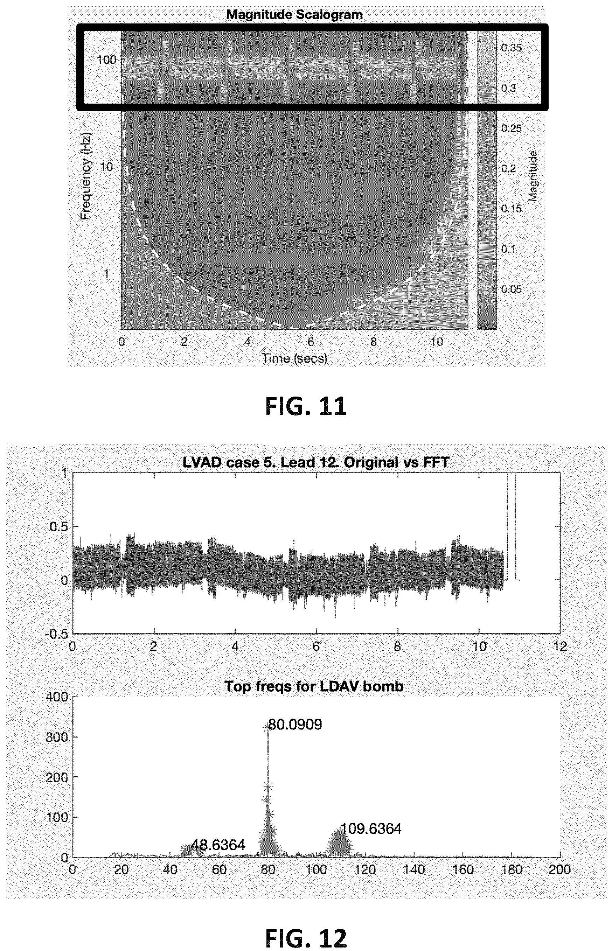

To do this, preferably a continuous Wavelet transform is performed (CWT) to the ECG data and a scalogram (time and frequency analysis) is obtained. Figure 11 represents the scalogram, wherein the heart peaks and also the noise coming from the pump are shown.

Then, a classifier is applied to the scalogram or similar time and frequency analysis tool to determine whether the ECG contains information on LVAD or not. The classifier could use deep learning techniques or image processing techniques.

If the presence of a LVAD is detected, a Fast Fourier Transform (FFT) is applied to transform the data into the frequency domain. Figure 12 shows an ECG (lead 12) and its corresponding representation after applying the FFT. If no external assistance devices are detected, the filtered ECG signal outputted is equal to the ECG signal obtained without filtering.

The signal should be filtered for eliminating an interference due to alternating current (50Hz or 60 Hz), whose frequency is previously known.

Then, a search of the principal rotation frequency of the pump of the LVAD is carried out. Preferably, the search is centered in an operating range designated by the manufacturer with a safety margin.

Normally, the LVAD pump operates at a nominal speed (principal rotation frequency), then, every two seconds brakes (reducing it speed by 2,000 rpm), accelerates (by 4,000 rpm) and reduces speed (by 2,000rpm) to return to the nominal speed.

For determining the secondary rotational speeds, a clustering analysis is performed. The maximum of each cluster could be selected as a representative value of the secondary rotational speeds.

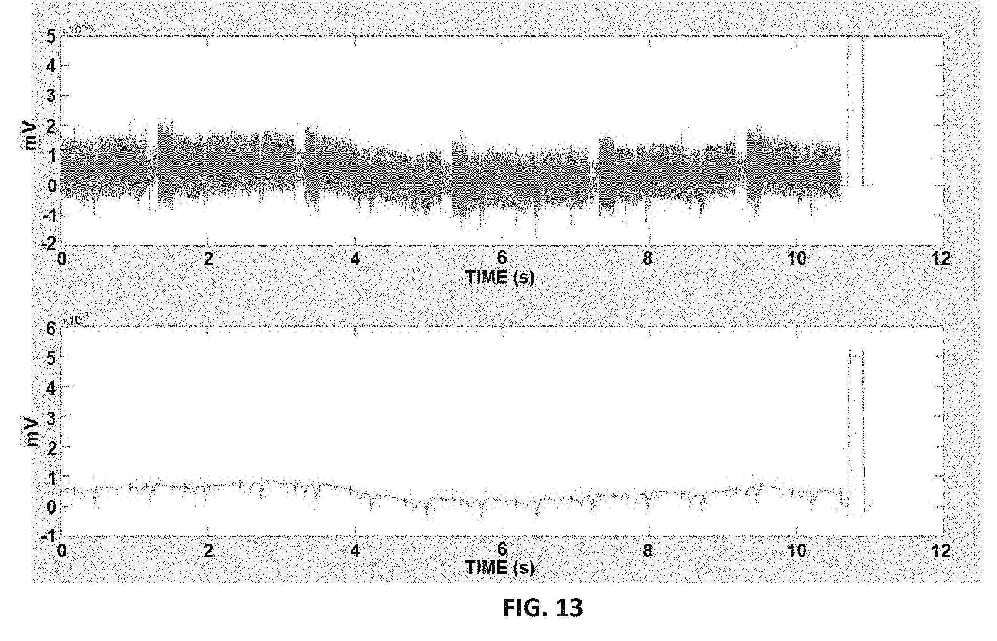

With the frequencies identified, a bank of Notch filters (or band elimination filters) is applied, resulting in filtered ECG data as shown in Figure 13. These filters are characterized by selective attenuation. The cut-off frequencies used in the example shown in Figure 11 are:
- Principal Rotation Frequency, in this case is at 80.0909 Hz, which is the main noise component. It is close to the value provided by the manufacturer, but it is not fixed. The specific principal rotation frequency is selected as a function of the patient.
- First Secondary Rotation Frequency, the maximum higher rotation in this case is at 109.6364 Hz.
- Alternate Current Interference Frequency, normally close to 50 or 60Hz.
- Second Secondary Rotation Frequency, the slowest rotation frequency, in this case is at 48.6364 Hz. A filter is not applied to this second secondary rotation frequency since it matches with natural heart frequencies. In case of extreme noise, this frequency could also be filtered, missing some patient's heart information.

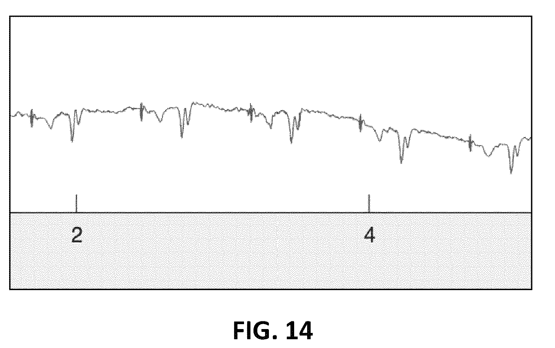

For precision filtering, the Notch filter is applied along with an IIR (infinite response filter) filter. In Figure 14, the ECG data corresponds to a patient having a pacemaker, despite the minimal amplitude of this patient's ECG, pacemaker's spicules are clearly visible in the filtered ECG data.

This filtered ECG can be post-processed with classic techniques (as a classical ECG).

As explained before, from the scalogram data, also the configuration of the pump of the LVAD can be inferred, such as rotation frequencies, time at each frequency, anomalies (e.g. not periodic operation), real rotation limits...

Then, the method of the invention outputs the ECG filtered, having minimal noise introduced by the LVAD, and pump operating characteristics during the obtention of the ECG data.

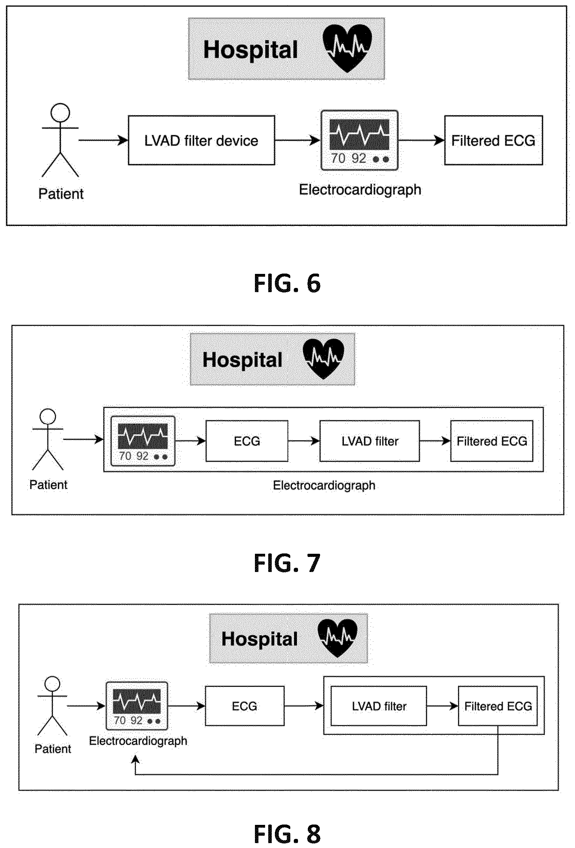

Depending on the use case, the output data could be a digital ECG signal or an analog ECG signal. When the method is applied by a processing module between electrodes, connected to the patient, and an ECG module, configured to digitalize and process ECG measurement, the method outputs an analog ECG signal to be processed by the ECG module. This configuration is shown in Figure 6.

When the method is applied by a processing module after the ECG module, configured to digitalize and process ECG measurement, the method outputs a digital ECG signal to be post-processed as a classical ECG. For this case, the processing module could be physically in the same device than the ECG module, as shown in Figure 7, in a separate device connected to the ECG module, as shown in Figure 8, or in a cloud server connected to the ECG module, as shown in Figure 9.

Thus, the method of the invention can be implemented as:
- a software solution configured to run on a processor capable of obtaining an ECG (electrocardiograph, mobile devices, cardiac monitoring implants, etc.);
- a system for post-processing an ECG in digital format in any of the available formats and standards (HL7, Dicom, FHIR, XML, csv, JSON, etc.); or
- a hardware solution configured to act as an active digital filter of the ECG module.

## Claims

1. A method for obtaining ECG data, comprising the steps of:
- obtaining ECG data from a patient;
- checking if the ECG data comprises noise patterns from a Left Ventricular Assist Device (LVAD), which comprises a pump, by analyzing the obtained ECG data using a time and frequency transformation and classifying the analyzed ECG data as with or without noise patterns, and
- if the ECG data comprises LVAD noise patterns:
∘ applying a frequency transformation for transforming the ECG data into a frequency domain;
∘ search for a principal rotation frequency of the pump in a range previously known;
∘ clustering the ECG data by frequency, generating one or more clusters;
∘ determining secondary rotation frequencies of the pump from clusters generated;
∘ filtering the principal rotation frequency and the secondary rotation frequencies from the ECG data; and
∘ outputting filtered ECG data
- if the ECG data does not comprise LVAD noise patterns:
∘ outputting the obtained ECG data as filtered ECG data.

2. The method according to claim 1, further comprising a step of filtering also an alternate current interference, with a previously known frequency.

3. The method according to any of previous claims, wherein the step of checking if the ECG data comprises noise patterns from the LVAD comprises the sub-steps of:
- analyzing the ECG data in frequency and time by performing a Continuous Wavelet Transform (CWT), thus, obtaining a scalogram; and
- classifying the analyzed ECG data for determining the presence of the LVAD.

4. The method according to claim 3, further comprising a step of determining and outputting actual operation information of the LVAD, being its working frequencies and time on each frequency.

5. The method according to claim 4, further comprising a step of determining anomalies in the LVAD by checking if the principal rotation frequency and the secondary rotation frequencies obtained corresponds to a range previously known.

6. The method according to claim 1, wherein the filtering step is performed by using sequentially dynamic filters.

7. The method according to claim 6, wherein the filtering step is performed by using Notch filters implemented with Infinite Impulse Response (IIR) filters.

8. The method according to claim 3, wherein the step of classifying the analyzed ECG data for determining the presence of a LVAD is performed using deep learning classification or using image processing techniques.

9. The method according to claim 1, wherein the step of applying a frequency transformation is performed using a Fast Fourier Transform (FFT).

10. The method according to claim 1, wherein the step of clustering the ECG data by frequency further comprises a stage of selecting a maximum value of each cluster as each secondary rotation frequency.

11. An ECG system comprising:
- one or more electrodes intended to be attached to a patient and configured to obtain ECG measurements;
- an ECG module, connected to the electrodes and configured to digitalize and process ECG measurements, and
- a processing module connected to the ECG module and configured to perform the steps of the method according to any of claims 1 to 10.

12. The ECG system according to claim 11, wherein the processing module is placed between the electrodes and the ECG module.

13. The ECG system according to claim 11, wherein the processing module is placed after the ECG module.

14. The ECG system according to claim 11, wherein the processing module is placed in a cloud server connected to the ECG module and configured to receive ECG data from said ECG module.

15. The ECG system according to claim 11, wherein the processing module is placed in a mobile device connected to the ECG module and configured to receive ECG data from said ECG module.
